# EUROPEAN PATENT SPECIFICATION

(11) **EP 0 258 598 B1**
(45) Date of publication and mention of the grant of the patent: **07.09.1994**
(21) Application number: 87110434.5
(22) Date of filing: 18.07.1987
(51) Int. Cl.: C07D 241/08, C07D 241/04, C07D 241/38, C07D 295/12, C07D 295/16, C07D 295/22, C08K 5/34, C08K 5/3462

(54) **N-(substituted-1-piperazinealkyl)-alpha-(3,5-di-alkyl-4-hydroxyphenyl)-alpha,alpha-dialkyl acetamides**
N-(substituiertes-1-Piperazinalkyl)-alpha-(3,5-dialkyl-4-hydroxyphenyl)-alpha,alpha-dialkylacetamide
Alpha-(3,5-dialkyl-4-hydroxyphényl)-alpha,alpha-dialkylacétamides N-substitués par pipérazine substituée

(30) Priority: 21.07.1986 US 887458
(43) Date of publication of application: 09.03.1988
(73) Proprietor: THE B.F. GOODRICH COMPANY, Akron Ohio 44313-1799 (US)
(72) Inventor: Lai, John Ta-Yuan, Broadview Heights Ohio 44147 (US); Son, Pyong-Nae, Akron Ohio 44313 (US)
(74) Representative: von Kreisler, Alek, Dipl.-Chem.

(56) References cited:
- EP-A- 1 284
- EP-A- 104 645
- EP-A- 0 112 802
- EP-A- 0 141 419
- EP-A- 0 253 010
- US-A- 4 477 665

## Description

### SUMMARY OF THE INVENTION

N-(substituted-1-(piperazine alkyl))-α-(3,5-di-alkyl-4-hydroxyphenyl)-α,α-dialkyl acetamides prepared by a new process are novel stabilizers for organic materials subject to attack and degradation by heat, oxygen and light, and form useful combinations for this purpose with other hindered phenol stabilizers.

US-A-4 477 665 discloses stabilizers for the stabilization of polymeric materials against ultraviolet light and the oxygen present in the environment. These stabilizers are of the formula: 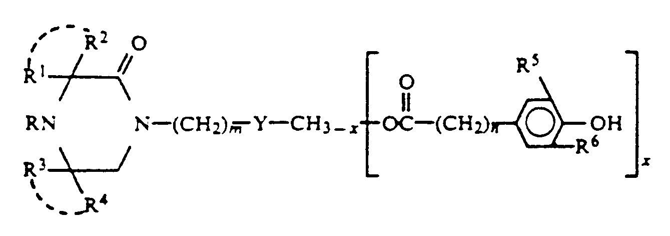
wherein R¹, R³ and R⁶ are independently selected from the group consisting of alkyl of 1-8 carbon atoms, aryl, cycloalkyl, and alkalene of 2-6 carbon atoms;
R² is alkyl of 1-6 carbon atoms and can, in conjunction with an acyclic substituent of R¹ form a cyclic substituent;
R⁴ is alkyl of 1-6 carbon atoms and can, in conjunction with an acyclic substituent of R³, form a cyclic substituent;
R⁵ is hydrogen or alkyl of 1-6 carbon atoms;
R is hydrogen or alkyl of 1-6 carbon atoms;
Y is oxygen, nitrogen or sulfur;
m is 0 to 6;
n is 0 to 3; and
X is 1 to 3.

EP-A-0 141 419 pertains to stabilizers for organic materials subject to attach and degradation by heat, oxygen and light.
In detail 3,5-dialkyl-4-hydroxyphenyl-substituted derivatives are disclosed having the formula 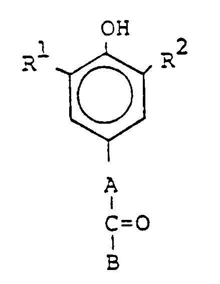
wherein R¹ and R² are alkyl and cycloalkyl groups containing 1 to 12 carbon atoms, alkylcycloalkyl and aryl or alkaryl groups wherein the alkyl groups contain 1 to 8 carbon atoms, and the aryl groups are normally phenyl or naphthyl; A is 1) an alkyl group of the formula 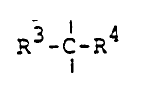
wherein R³ and R⁴ are alkyl groups containing 1 to 18 carbon atoms, C₁-C₁₈, or; 2) a cycloalkyl group containing 5 to 12 carbon atoms and alkaryl groups wherein the alkyl groups contain 1 to 8 carbon atoms; B is OH, 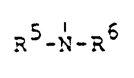
or -O-R⁷, wherein R⁵ and R⁶ are hydrogen, alkyl, aryl, hydroxyalkyl, cycloalkyl and arylalkyl radicals containing from 1 to about 18 carbon atoms, and R⁷ is an alkyl, alkylidene, cycloalkyl, or alkaryl radical containing 1 to 18 carbon atoms.
It was therefore an object of the present invention to provide further acetamide compounds exhibiting oxidative stabilization properties.

### DETAILED DESCRIPTION

The novel N-(substituted-1-(piperazine alkyl)) -α-(3,5-di-alkyl-4-hydroxyphenyl)-α,α-dialkyl acetamides of this invention have the general formula 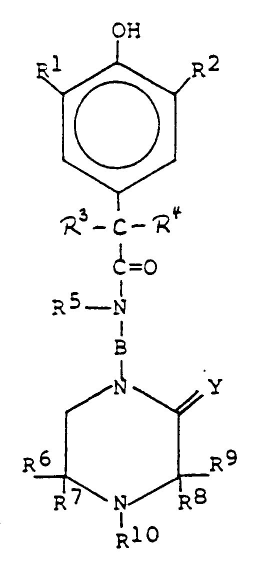
wherein R¹ and R² are alkyl groups containing 1 to 12 carbon atoms,
R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms, R⁶ and R⁷, R⁸ and R⁹ can be or form rings containing 5 to 7 carbon atoms;
R³ and R⁴ are alkyl groups containing 1 to 18 carbon atoms;
B is an alkylidene group of the formula 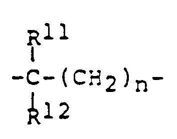
wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 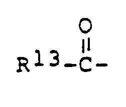
wherein R¹³ is hydrogen, alkyl or alkenyl containing up to 18 carbon atoms, phenyl or naphthyl, O or OH; and Y is H₂ or O, provided that R¹¹ and R¹² are not both hydrogen.

Preferably, R¹ is alkyl containing 1 to 8 carbon atoms, R² is alkyl containing 1 to 6 carbon atoms, and more preferably at least one of R¹ and R² is a t-alkyl group, including t-butyl and t-amyl; R³ and R⁴ are alkyl groups containing 1 to 8 carbon atoms, more preferably 1 to 4 carbon atoms; R⁵ is hydrogen, alkyl containing 1 to 6 carbon atoms; R⁶ and R⁷ and R⁸ and R⁹ are alkyl containing 1 to 6 carbon atoms or rings containing 5 to 7 carbon atoms; R¹⁰ is hydrogen; in B, n is 1 to 3 and R¹¹ and R¹² are hydrogen or methyl; provided that R¹¹ and R¹² are not both hydrogen.

These N-(substituted-1-(piperazine alkyl)) -α-(3,5-di-alkyl-4-hydroxyphenyl)-α,α-dialkyl acetamides, more specifically N-alkyl-N-(3,3,5,5-tetraalkyl -2-keto-1-piperazinealkyl)-α-(3,5-di-alkyl-4-hydroxyphenyl)-α,α-dialkylacetamides, are prepared by a new process by reacting a 2,6-di-alkylphenol with an aliphatic ketone (for compounds of claim 1) or cyclohexanone (for compounds of claim 6), a haloform and a piperazine compound in the presence of alkali metal hydroxide. An organic solvent may be used, or large amounts of the ketone reactant may be employed. The amide is isolated in excellent yields by crystallizing the filtered reaction product.

Typical 2,6-dialkyl phenols used have the formula 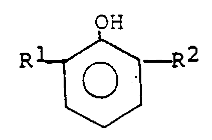
wherein R¹ and R² have the meanings set forth above, including 2-methyl-6-t-butylphenol, 2-ethyl-6-t-butylphenol, 2-propyl-6-t-butylphenol, 2-isopropyl-6-t-butylphenol, 2-n-butyl-6-t-butylphenol, 2,6-di-t-butylphenol, 2-n-amyl-6-t-butylphenol, 2-isoamyl-6-t-butylphenol, 2-hexyl-6-t-butylphenol, 2-heptyl-6-t-butylphenol, 2-isooctyl-6-t-butylphenol, 2-isopropyl-6-methylphenol, 2-n-butyl-6-isopropylphenol, 2-isoamyl-6-ethylphenol, 2-isoamyl-6-methylphenol, 2-isooctyl-6-methylphenol, 2-isooctyl-6-ethylphenol, 2-isooctyl-6-n-propylphenol, 2-isooctyl-6-n-hexylphenol.

The ketones used include dialkyl ketones, and cyclohexanone.

Typical ketones that may be used in the novel process to make the new 3,5-dialkyl-4-hydroxyphenyl-substituted acetic acids of the invention are alkyl ketones of the formula 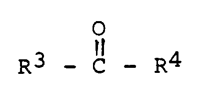
wherein R³ and R⁴ are alkyl radicals containing 1 to 18 carbon atoms, preferably 1 to 8 carbon atoms, C₁-C₈, including for example acetone, methyl ethyl ketone, methyl n-propyl ketone, diethyl ketone, 2-hexanone, 3-hexanone, di-n-propyl ketone, 2-octanone, methyl isopropyl ketone ; in amounts from at least 1 mole of ketone per mole of 2,6-dialkylphenol, up to amounts sufficient for the ketones to be the solvent of the reaction, 10 moles or more. No more than two moles is preferred when the ketone is a reactant only. Use of less than 1 mole reduces the product yield, and more than 2 moles is unnecessary unless the ketone is the solvent.

The haloform, such as chloroform, is also used in a molar ratio of at least about one mole per mole of 2,6-dialkylphenol used, but preferably a slight molar excess is used up to about a 50 percent molar excess, i.e., 1.5 mole per mole of 2,6-dialkylphenol. While larger amounts may be used, there is no advantage realized, and lesser amounts will decrease the ultimate yield of the desired product. Bromoform may be substituted for the chloroform and excellent results will be obtained.

The alkali metal hydroxide is used in powder form, or in solution, and includes sodium hydroxide and potassium hydroxide. Preferably, a molar excess of the alkali metal hydroxide is used in relation to the amount of 2,6-dialkylphenol present. Normally from 4 moles of alkali metal hydroxide per mole of 2,6-dialkylphenol up to 10 or more moles may be used, but the amount used preferably is 4 to 8 moles of hydroxide per mole of 2,6-dialkylphenol. Use of less than 4 moles will reduce the yield of desired product.

The N-substituted-1-(piperazine alkyl) used in the process of this invention to make the novel acetamides, have the general formula 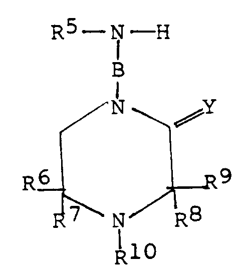
wherein R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms,
and R⁶ and R⁷, R⁸ and R⁹ can be or form rings containing 5 to 7 carbon atoms; B is an alkylidene group of the formula 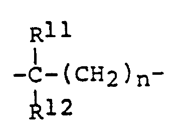
wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 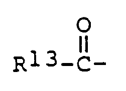
wherein R¹³ is hydrogen, alkyl or alkenyl containing up to 18 carbon atoms, phenyl or naphthyl, O or OH; and Y is H₂ or O, provided that R¹¹ and R¹² are not both hydrogen.

More preferably B is an alkylidene group of the formula 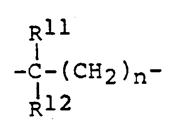
wherein n is 1 to 8, preferably 1 to 3, R¹¹ and R¹² are H or alkyl groups of 1 to 8 carbon atoms, preferably H and methyl. Typical compounds are substituted piperazine derivatives such as N-(2-isopropylaminoethyl)-3,3,5,5-tetramethyl-2-piperazinone, N-(2-cyclohexylaminoethyl)-3,3,5,5-tetramethyl-2-piperazinone, N-(2-isobutylaminoethyl)-3,5,5-trimethyl-3-isobutyl-2-piperazinone.

The amount of substituted piperazine used in the reaction is based on one mole of the 2,6-dialkylphenol. While the amount may vary from less than a stoichiometric amount, resulting in low product yield, to larger excesses that are not required and have to be separated from the reaction mixture, normally 1 to 4 moles to 1 mole is used.

The solvent used may be any polar organic solvent, including an excess of the ketone used in place of an added solvent. Typical solvents that may be used include methylene chloride, tetrahydrofuran, diethyl ether, dibutyl ether, dimethyl sulfoxide, 1,4-dioxane, carbon tetrachloride, toluene, xylene. The amounts of solvent used will vary from about 5 moles to 100 moles per mole of 2,6-dialkylphenol used. As has been stated, the solvent may be eliminated if an excess of the reactant ketone is used. In this case, the amount of ketone used, based on the moles of 2,6-dialkylphenol used may be from 5 to 20, preferably 7.5 to 15 moles per mole of 2,6-dialkylphenol.

While the reactants may be added in any order, it is preferred that the alkali metal hydroxide be added last, over a period of time to control the exothermic reaction and preferably maintain the reaction below 30°C, preferably below 10°C. The reaction temperature may be varied from 0°C to 30°C, but preferably is conducted from 0°C to 10°C. The reaction time normally will vary from 5 to 15 hours.

The amides are readily isolated from the reaction mixture by filtration, washing the filtrate with aqueous inorganic acid, including hydrochloric or sulfuric acid, to the reaction mixture to isolate the amide, most of which is in the organic layer that forms. The aqueous layer may be extracted with solvent to remove all traces of the amide, and this is added to the other organic layer and this mixture is dried with a desiccant such as anhydrous sodium sulfate, and heated to dryness. This product may be recrystallized if desired.

The practice of the invention is demonstrated in the following Examples. The structures of the amides of the following Examples were confirmed by infrared and nuclear magnetic resonance spectra. Molecular weights were determined and confirmed by field desorption mass spectra (FD/MS). Elemental analysis of carbon and hydrogen was done and the amounts found were consistent with the formulas of the materials.

### Example 1

### N-[1-(2-keto-3,3,5,5-tetramethyl-1-piperazinyl)-2-methyl 2-propyl]-2-(3,5-di-t-butyl-4-hydroxyphenyl)-2-methyl-propanamide

0.1 mole of Imino-bis-(2-amino-2-methyl-1-propane) 0.15 mole, chloroform and 2.0 moles of acetone were placed in a 3-neck flask equipped with a thermometer, a mechanical stirrer, cooling means and an addition funnel. The mixture was kept cool with a circulating bath, while 0.5 mole of sodium hydroxide beads were added in small portions to keep the reaction temperature below 10°C. After the addition, the reaction was stirred under nitrogen until gas chromatograph showed the reaction of the amine was substantially complete. 0.14 moles of Chloroform and 0.1 mole of 2.6-di-t-butylphenol were added, followed by 0.5 mole of sodium hydroxide beads in portions to keep the temperature below 10°c. The mixture was stirred at 10°C overnight. The mass was concentrated in a rotary evaporator to remove solvents and low boilers and stirred in a hexanes-water mixture. The solid was collected and washed with hexanes and water. The yield was about 70% after drying. The propanamide was recrystallized from 70% ethanol with a few drops of 85% hydrazine to yield white crystals. The melting point was 145-8°C.

### Example 2

### N-[1-(2-keto-3,5,5-trimethyl-3-ethyl-1-piperazinyl)-2-methyl-2-propyl]-2-(3,5-di-t-butyl-4-hydroxyphenyl)-2-methyl-butanamide

0.1 mole of imino-bis-(2-amino-2-methyl-1-propane) 0.15 mole of chloroform and 2.0 moles of 2-butanone were placed in a 3-neck flask equipped with a thermometer, a mechanical stirrer, and an addition funnel. While cooling with a circulating bath, 0.5 mole of sodium hydroxide beads were added in small portions to keep the reaction temperature below 10°C. After the reaction was complete, 0.14 mole of Chloroform and 0.1 mole of 2.6-di-t-butylphenol was added, followed by 0.5 mole of sodium hydroxide beads. The recrystallized butaneamide had a melting point of 126-8°C.

### Example 3

### N-[1-(2-keto-3,3 pentamethylene-5,5-dimethyl-1-piperazinyl)-2-methyl-2-propyl]-α-(3,5-di-t-butyl-4-hydroxyphenyl)-cyclohexanecarboxamide

0.1 mole of imino-bis-(2-amino-2-methyl-1-propane) 0.15 mole of chloroform and 2.0 moles of cyclohexanone were placed in a 3-neck flask and while cooling with a circulating bath, 0.5 moles sodium hydroxide beads were added in small portions to keep the reaction temperature below 10°C. After the reaction was complete, 0.14 mole of Chloroform and 0.1 mole of 2.6-di-t-butylphenol were added, followed by sodium hydroxide beads. The recrystallized cyclohexanecarboxamide had a melting point of 179-182°C.

### Example 4

### N-[1-(2-keto-3,3,5,5-tetramethyl-1-piperazinyl)-2-methyl-2-propyl]-α-(3,5-di-t-butyl-4-hydroxyphenyl) cyclohexanecarboxamide

0.1 mole of N-(2-amino-2-methyl-1-propyl)-3, 3,5,5-tetramethyl-2-piperazine, 0.2 mole of chloroform, 2.0 mole of cyclohexanone and 0.2 mole 2.6-di-t-butylphenol were mixed and cooled at about 5°C while 0.5 mole of sodium hydroxide beads were added in small portions to keep the temperature below 10°C. The mixture was stirred at 10°C overnight and the mixture was concentrated to remove most cyclohexanone and 2.6-di-t butylphenol. This product was stirred in water or water-hexanes mixture to obtain a solid. This solid was recrystallized from hexanes to yield an off-white colored powder. The melting point was 125-130°C.

### Example 5

### N-Cyclohexyl-N[2-(2-keto-3,3,5,5-tetramethyl-1-piperazinyl ethyl]-α-(3,5-di-t-butyl-4-hydroxyphenyl) cyclohexanecarboxamide

0.1 mole of N-(2 cyclohexylaminoethyl)-3,3, 5,5-tetramethyl-2-piperazinone, 0.2 mole of chloroform, 2.0 mole of cyclohexanone and 0.2 mole 2.6-di-t-butylphenol were mixed and cooled at about 5°C while 0.5 mole of sodium hydroxide beads were added in small portions to keep the temperature below 10°C. The cyclohexane carboxamide had a melting point of about 60°C.

### Example 6

### N-Cyclohexyl-N-[3-(2-keto-3,3,5,5-tetramethyl-1-pipiperazinyl propyl]-α-(3,5-di-t-butyl-4-hydroxyphenyl) cyclohexanecarboxamide

0.1 mole of N-(3-cyclohexylamino-1-propyl)-3,3,5,5-tetramethyl-2-piperazinone, 0.2 mole of chloroform, 2.0 mole of cyclohexanone and 0.2 mole of 2.6-di-t-butylphenol were mixed and cooled at about 5°C while 0.5 mole of sodium hydroxide beads were added in small portions to keep the temperature below 10°C. The cyclohexanecarboxamide had a melting point of about 60°C.

Organic materials which can be stabilized in accordance with the present invention include both natural and synthetic polymers. For example, the stabilizers are useful for the stabilization of cellulosic materials; natural rubber, halogenated rubber, conjugated diene polymers, as for instance, polybutadiene, copolymers of butadiene with styrene, acrylonitrile, acrylic acid, alkyl acrylates or methacrylates, methyl vinyl ketone, vinyl pyridine; polyisoprene, polychloroprene; vinyl polymers such as polyvinyl chloride, polyvinylidene chloride, copolymers of vinyl chloride with vinylidene chloride, polyvinyl acetate, copolymers or vinyl halide with butadiene, styrene, vinyl esters, α,β-unsaturated ketones and aldehydes; homopolymers and copolymers of acrylic moonomers such a methyl acrylate, methyl methacrylate, ethyl acrylate, 3-ethylhexyl acrylate, acrylamide, methacrylamide, N-methylol-acrylamide, acrylonitrile, methacrylonitrile; ephihalohydrin polymers; polyether- or polyol-derived polyurethanes; acetal homo-polymers and copolymers; polycarbonates; polyesters such as those derived from maleic, fumaric, itaconic, or terephthalic anhydrides, for example, polyethylene terephthalate; polyamides such as those derived from the reaction of hexa-methylenediamine with adipic or sebacic acid; epoxy resins such as those obtained from the condensation of epichlorohydrin with bisphenols; ring opened olefin polymers. Polymer blends, that is, physical admixture of two or more polymers may also be stabilized in accordance with the present invention.

In addition to polymeric materials, the present compounds may stabilize a wide variety of other organic materials. Such compounds include: waxes, synthetic and petroleum-derived lubricating oils and greases; animal oils such as, for example, fat, tallow, lard, cod-liver oil, sperm oil; vegetable oils such as castor, linseed, peanut, palm, cotton seed, and the like; fuel oil; diesel oil, gasoline.

The N-(substituted-1-(piperazine alkyl))-α-(3,5-di-alkyl-4-hydroxyphenyl)-α',α''dialkyl acetamides as defined herein provide exceptional heat stability and resistance to ultraviolet degradation to polyolefin polymers. They are especially useful for the stabilization of α-monoolefin homopolymers and copolymers, wherein the α-monoolefin contains 2 to 8 carbon atoms. High and low-density polyethylene, isotactic and atactic polypropylene, polyisobutylene, and poly(4-methyl-1-pentene) have excellent resistance to heat and oxygen when stabilized with the combinations of the present invention. Ethylene-propylene copolymers and ethylene-propylene terpolymers, generally containing less than 10 percent by weight of one or more monomers containing multiple unsaturatation provided, phenols; ring opened olefin polymers. Polymer blends, that is, physical admixture of two or more polymers may also be stabilized in accordance with the present invention.

The (3,5-dialkyl-4-hydroxyphenyl)-substituted amides may be used with other stabilizers including hindered or partially hindered phenols, hindered amine light stabilizers, phosphites, o-hydroxybenzophenones. Typical hindered phenols are the hydroxyphenylalkyl isocyanurates such as the symmetrical tris(3,5-di-t-alkyl-4-hydroxybenzyl) isocyanurates; tetrakis[methylene 3-(3',5'-dialkyl-4'-hydroxyphenyl)propanoate] methanes wherein the alkyl groups contain 1 to 8 carbon atoms, such as tetrakis[methylene 3-(3',5'-di-t-butyl-4'-hydroxyphenyl)propanoate]methane; alkyl (3-3',5'-di-t-butyl-4'-hydroxyphenyl) propionates wherein the alkyl groups contain 1 to 18 carbon atoms, such as octadecyl 3-(3',5'-di-t-butyl-4-hydroxyphenyl)propionate; 1,3,5-trimethyl-2,4,6-tris[3,5-dialkyl-4-hydroxybenzyl)benzene; 1,3,5-tris(3,5-di-t-butyl-4-hydroxyhydrocinnamoylethyl)-s-triazine-2,4,6-(1H,3H,5H)-trione; 2,2'-alkylidene bis (4,6-dialkylphenol)-s wherein the alkyl group contains 1 to 8 carbon atoms, such as 2,2'-methylene bis(4,6-di-t-butylphenol), 2,2'-ethylidene bis(4,6-di-t-butylphenol), and 2,2'-methylene bis(4-methyl-6-t-butylphenol).

Additional test samples of the defined acetamides in polypropylene were prepared by combining 0.2 weight part of the hereinafter described acetamide, 0.1 weight part of calcium stearate processing acid and 0.1 weight part of a commercial stabilizer, tris (3, 5-di-t-butyl-4-hyroxy-benzyl)isocyanurate, with 100 weight parts of polypropylene. The resulting mixtures were pelletized and fibers spun therefrom. The fibers were tested in the Weatherometer® as described herein above and the hours to failure determined. For a sample with the tris (3, 5-di-t-butyl-4-hyroxy-benzyl)isocyanurate, the hours to failure were 260.

With N-[1-(2-keto-3,3,5,5-tetramethyl-1-piperazinyl)-2-methyl-2-propyl]-2-(3,5-di-t-butyl-4-hydroxyphenyl)-2-methyl-propanamide, 1,080 hours.

With N-[1-(2-keto-3,3-pentamethylene-5,5'-di-methyl-1-piperazinyl)-2-methyl-2-propyl]-α-(3,5-di-t-butyl-4-hydroxyphenyl) cyclohexanecarboxamide, 980 hours, and
With N-cyclohexyl-N-[3-(2-keto-3,3,5,5'-tetramethyl-1-piperazinyl)-2-methyl-2-propyl]-2-(3,5-di-t-butyl-4-hydroxyphenyl)-2-methyl propioamide, 530 hours to failure.

## Claims (Claims for the following Contracting State(s): BE, CH, DE, FR, GB, GR, IT, LI, LU, NL, SE)

1. N-(substituted)-1-(piperazine alkyl)-α-(3,5-di-alkyl-4-hydroxyphenyl)-α,α-dialkyl acetamides having the general formula 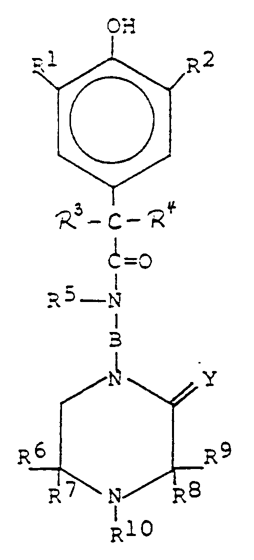 wherein R¹ and R² are alkyl groups containing 1 to 12 carbon atoms,
R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms, R⁶ and R⁷, R⁸ and R⁹ can be or form rings containing 5 to 7 carbon atoms;
R³ and R⁴ are alkyl groups containing 1 to 18 carbon atoms;
B is an alkylidene group of the formula 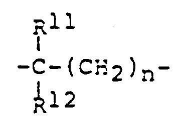 wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 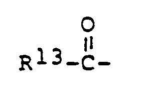 wherein R¹³ is hydrogen, alkyl or alkenyl containing up to 18 carbon atoms, phenyl or naphthyl, O or OH; and Y is H₂ or O, provided that R¹¹ and R¹² are not both hydrogen.

2. Compounds of Claim 1, wherein R¹ is alkyl containing 1 to 8 carbon atoms, R² is alkyl containing 1 to 6 carbon atoms, and at least one of R¹ and R² is a t-alkyl group, R³ and R⁴ are alkyl groups containing 1 to 8 carbon atoms;
R⁵ is hydrogen or alkyl containing 1 to 6 carbon atoms; R⁶ and R⁷ and R⁸ and R⁹ are alkyl containing 1 to 6 carbon atoms or rings containing 5 to 7 carbon atoms; in B, n is 1 to 3 and R¹¹ and R¹² are hydrogen or methyl; R¹⁰ is hydrogen or alkyl radicals containing 1 to 18 carbon atoms, O or OH; and Y is H₂ or O, provided that R¹¹ and R¹² are not both hydrogen.

3. N-alkyl-N-(3,3,5,5-tetraalkyl-2-keto-1-piperazinyl)-α-(3,5-di-alkyl-4-hydroxyphenyl-α,α -dialkylacetamides of Claim 2 wherein R³ and R⁴ contain 1 to 4 carbon atoms.

4. The acetamides of Claim 2 wherein at least one of R¹ and R² is t-butyl or t-amyl.

5. Compounds according to claims 1 to 4 selected from
N-[1-(2-keto-3,3,5,5-tetramethyl-1-piperazinyl)-2-methyl-2-propyl]-α-(3,5-di-t-butyl-4-hydroxyphenyl)-2-methyl-propanamide.
N-[1-(2-keto-3,5,5-trimethyl-3-ethyl-1-piperazinyl)-2-methyl-2-propyl]-α-(3,5-di-t-butyl-4-hydroxyphenyl)-2-methyl-propanamide.

6. Compounds selected from N-[1-(2-keto-3,3-pentamethylene-5,5-di-methyl-1-piperazinyl)-2-methyl-2-propyl]-α-(3,5-di-t-butyl-4-hydroxyphenyl)cyclohexanecarboxamide;
N-[1-(2-keto-3,3,5,5-tetramethyl-1-piperazinyl)-2-methyl-2-propyl]-α-(3,5-di-t-butyl-4-hydroxyphenyl)cyclohexanecarboxamide;
N-cyclohexyl-N-[2-(2-keto-3,3,5,5-tetramethyl-1-piperazinyl)ethyl]-α-(3,5-di-t-butyl-4-hydroxyphenyl)cyclohexanecarboxamide;
N-cyclohexyl-N-[3-(2-keto-3,3,5,5-tetramethyl-1-piperazinyl)propyl]-α-(3,5-di-t-butyl-4-hydroxyphenyl)cyclohexanecarboxamide.

7. A composition comprising (1) organic materials subject to degradation and stabilizing amounts of (2) compounds according to claims 1 to 6.

8. Composition of Claim 7 wherein (1) said solid organic material is a polymer and (2) is selected from the group consisting of
N-[1-(2-keto-3,3, 5,5-tetramethyl-1-piperazinyl)-2-methyl-2-propyl]-α-(3, 5-di-t-butyl-4-hydroxyphenyl)-2-methyl-propanamide; N-[1-(2-keto-3,5,5-trimethyl-3-ethyl-piperazinyl)-2-methyl-2-propyl]-α-(3,5-di-t-butyl-4-hydroxyphenyl)-2-methyl-propanamide; N-[1-(2-keto-3,3-pentamethylene-5, 5-di-methyl-1-piperazinyl)-2-methyl-2-propyl]-α-(3,5-di-t-butyl-4-hydroxyphenyl)cyclohexanecarboxamide; N-[1-(2-keto-3,3,5,5-tetramethyl-1-piperazinyl)-2-methyl -2-propyl]-(3,5-di-t-butyl-4-hydroxyphenyl)cyclohexane carboxamide; N-cyclohexyl-N-[2-(2-keto-3,3,5,5-tetramethyl-1-piperazinyl)ethyl]-α-(3,5-di-t-butyl-4-hydroxyphenyl)cyclohexanecarboxamide; N-cyclohexyl-N-[3-(2-keto-3,3,5,5-tetramethyl-1-piperazinyl)propyl] -α-(3,5-di-t-butyl-4-hydroxyphenyl)cyclohexanecarboxamide.

9. Composition of Claim 8 wherein the polymer is an olefin polymer.

10. A process for preparing N-(substituted)-1-(piperazine alkyl)-α-(3,5-di-alkyl-4-hydroxyphenyl)-α,α-dialkyl acetamides having the general formula 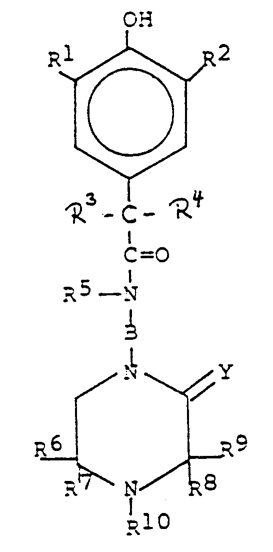 wherein R¹ and R² are alkyl groups containing 1 to 12 carbon atoms,
R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms, R⁶ and R⁷, R⁸ and R⁹ can be or form rings containing 5 to 7 carbon atoms;
R³ and R⁴ are alkyl groups containing 1 to 18 carbon atoms;
B is an alkylidene group of the formula 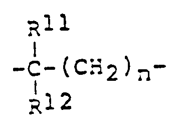 wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 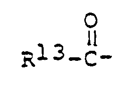 wherein R¹³ is hydrogen, alkyl or alkenyl containing up to 18 carbon atoms, phenyl or naphthyl, O or OH; and Y is H₂ or O provided that R¹¹ and R¹² are not both hydrogen; comprising reacting together a 2,6-dialkylphenol having the formula 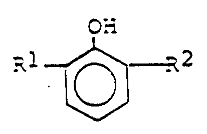 wherein R¹ andR² are alkyl groups containing up to 12 carbon atoms; a ketone selected from the group consisting of dialkyl ketones,
wherein the alkyl groups contain 1 to 18 carbon atoms, a haloform selected from the group consisting of chloroform and bromoform, and an alkali metal hydroxide and piperazines, having the general formula 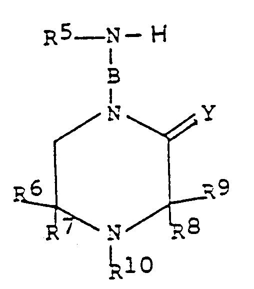 wherein R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms, and R⁶ and R⁷, R⁸ and R⁹ can be or form rings containing 5 to 7 carbon atoms; B is an alkylidene group of the formula 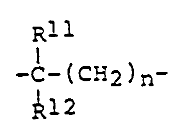 wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 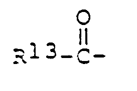 wherein R¹³ is hydrogen, alkyl or alkenyl containing up to 18 carbon atoms, phenyl or naphthyl, O or OH; and Y is H₂ or O, provided that R¹¹ and R¹² are not both hydrogen.

11. A process of Claim 10 wherein R¹ contains 1 to 8 carbon atoms and R² contains 1 to 6 carbon atoms, the ketone is a dialkyl ketone of the formula 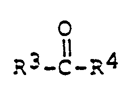 wherein R³ and R⁴ are alkyl radicals containing 1 to 8 carbon atoms;
R⁵ is hydrogen or an alkyl of 1 to 6 carbon atoms, R⁶ and R⁷ and R⁸ and R⁹ contain 1 to 8 carbon atoms; R¹⁰ is hydrogen; in B n is 1to 3 and R¹¹ and R¹² are hydrogen or methyl and Y is oxygen; provided that R¹¹ and R¹² are not both hydrogen.

## Claims (Claims for the following Contracting State(s): AT, ES)

1. A process for preparing N-(substituted)-1-(piperazine alkyl) -α-(3,5-di-alkyl-4-hydroxyphenyl)-α,α-dialkyl acetamides having the general formula 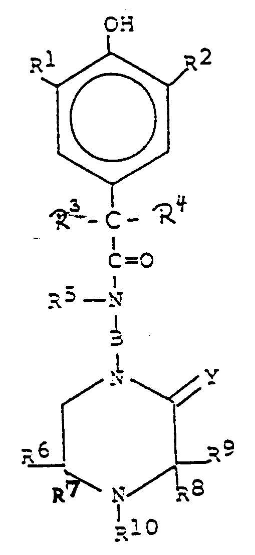 wherein R¹ and R² are alkyl groups containing 1 to 12 carbon atoms,
R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms, R⁶ and R⁷, R⁸ and R⁹ can be or form rings containing 5 to 7 carbon atoms;
R³ and R⁴ are alkyl groups containing 1 to 18 carbon atoms;
B is an alkylidene group of the formula 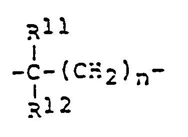 wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 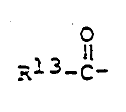 wherein R¹³ is hydrogen, alkyl or alkenyl containing up to 18 carbon atoms, phenyl or naphthyl, O or OH; and Y is H₂ or O provided that R¹¹ and R¹² are not both hydrogen; comprising reacting together 2,6-dialkylphenol having the formula 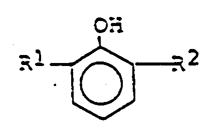 wherein R¹ andR² are alkyl groups containing up to 12 carbon atoms; a ketone selected from the group consisting of dialkyl ketones,
wherein the alkyl groups contain 1 to 18 carbon atoms, a haloform selected from the group consisting of chloroform and bromoform, and an alkali metal hydroxide and piperazines, having the general formula 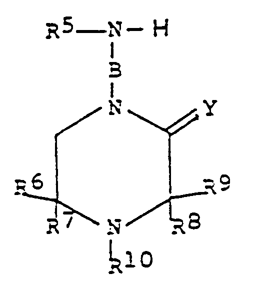 wherein R⁵, R⁶, R⁷, R⁸ and R⁹ are hydrogen, alkyl groups containing 1 to 12 carbon atoms, phenyl and naphthyl groups, cycloalkyl groups containing 4 to 12 carbon atoms, and R⁶ and R⁷, R⁸ and R⁹ can be or form rings containing 5 to 7 carbon atoms; B is an alkylidene group of the formula 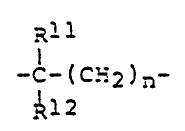 wherein n is 1 to 8, R¹¹ and R¹² are hydrogen or alkyl groups containing 1 to 8 carbon atoms; R¹⁰ is hydrogen, alkyl radicals containing 1 to 18 carbon atoms, acyl 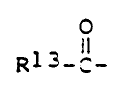 wherein R¹³ is hydrogen, alkyl or alkenyl containing up to 18 carbon atoms, phenyl or naphthyl, O or OH; and Y is H₂ or O, provided that R¹¹ and R¹² are not both hydrogen.

2. A process of Claim 1 wherein R¹ contains 1 to 8 carbon atoms and R² contains 1 to 6 carbon atoms, the ketone is a dialkyl ketone of the formula 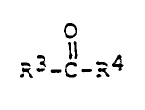 wherein R³ and R⁴ are alkyl radicals containing 1 to 8 carbon atoms;
R⁵ is hydrogen or an alkyl of 1 to 6 carbon atoms, R⁶ and R⁷ and R⁸ and R⁹ contain 1 to 8 carbon atoms; R¹⁰ is hydrogen; in B n is 1to 3 and R¹¹ and R¹² are hydrogen or methyl and Y is oxygen; provided that R¹¹ and R¹² are not both hydrogen.

3. A process of claim 2 wherein R³ and R⁴ contain 1 to 4 carbon atoms.

4. A process of Claim 2 wherein at least one of R¹ and R² is t-butyl or t-amyl.

5. A process according to claims 1 to 4 for preparing compounds selected from
N-[1-(2-keto-3,3,5,5-tetramethyl-1-piperazinyl)-2-methyl-2-propyl]-α-(3,5-di-t-butyl-4-hydroxyphenyl)-2-methyl-propanamide.
N-[1-(2-keto-3,5,5-trimethyl-3-ethyl-1-piperazinyl)-2-methyl-2-propyl]-α-(3,5-di-t-butyl-4-hydroxyphenyl)-2-methyl-propanamide.

6. A process according to claims 1 to 4 for preparing compounds selected from N-[1-(2-keto-3,3-pentamethylene-5,5-dimethyl-1-piperazinyl)-2-methyl-2-propyl]-α-(3,5-di-t-butyl-4-hydroxyphenyl)cyclohexancarboxamide;
N-[1-(2-keto-3,3,5,5-tetramethyl-1-piperazinyl)-2-methyl-2-propyl]-α-(3,5-di-t-butyl-4-hydroxyphenyl)cyclohexancarboxamide;
N-Cyclohexyl-N-[2-(2-keto-3,3,5,5-tetramethyl-1-piperazinyl)ethyl]-α-(3,5-di-t-butyl-4-hydroxyphenyl)cyclohexancarboxamide;
N-Cyclohexyl-N-[3-(2-keto-3,3,5,5-tetramethyl-1-piperazinyl)propyl]-α-(3,5-di-t-butyl-4-hydroxyphenyl)cyclohexancarboxamide, whereby the dialkyl ketone is substituted by cyclohexanone.

7. A composition comprising (1) organic materials subject to degradation and stabilizing amounts of (2) compounds obtainable according to claims 1 to 6.

8. Composition of Claim 7 wherein (1) said solid organic material is a polymer and (2) is selected from the group consisting of N-[1-(2-keto-3,3,5,5-tetramethyl-1-piperazinyl)-2-methyl-2-propyl]-α-(3,5-di-t-butyl-4-hydroxyphenyl)-2-methyl-propanamide; N-[1-(2-keto-3,5,5-trimethyl-3-ethyl-piperazinyl)-2-methyl-2-propyl]-α-(3,5-di-t-butyl-4-hydroxyphenyl)-2-methyl-propanamide; N-[1-(2-keto-3,3-pentamethylene-5,5-di-methyl-1-piperazinyl)-2-methyl-2-propyl]-α-(3,5-di-t-butyl-4-hydroxyphenyl)cyclohexancarboxamide; N-[1-(2-keto-3,3,5,5-tetramethyl-1-piperazinyl-2-methyl-2-propyl]-(3,5-di-t-butyl-4-hydroxyphenyl)cyclohexane carboxamide; N-cyclohexyl-N-[2-(2-keto-3,3,5,5-tetramethyl-1-piperazinyl)ethyl]-α-(3,5-di-t-butyl-4-hydroxyphenyl)cyclohexancarboxamide; N-cyclohexyl-N-[3-(2-keto-3,3,5,5-tetramethyl-1-piperazinyl) propyl] -α-(3,5-di-t-butyl-4-hydroxyphenyl)cyclohexanecarboxamide.

9. Composition of Claim 8 wherein the polymer is an olefin polymer.

## Patentansprüche (Patentansprüche für folgende(n) Vertragsstaat(en): BE, CH, DE, FR, GB, GR, IT, LI, LU, NL, SE)

1. N-(substituierte)-1-(Piperazinalkyl)-α-(3,5-dialkyl-4-hydroxyphenyl)-α,α-dialkylacetamide der allgemeinen Formel 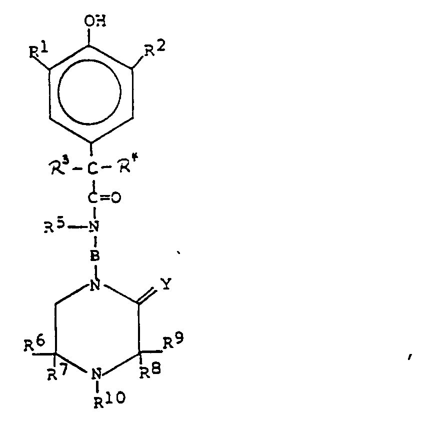 worin
R¹ und R² Alkyl-Gruppen mit 1 bis 12 Kohlenstoff-Atomen sind,
R⁵, R⁶, R⁷, R⁸ und R⁹ Wasserstoff, Alkyl-Gruppen mit 1 bis 12 Kohlenstoff-Atomen, Phenyl- und Naphthyl-Gruppen, Cycloalkyl-Gruppen mit 4 bis 12 Kohlenstoff-Atomen sind,
R⁶ und R⁷, R⁸ und R⁹ Ringe mit 5 bis 7 Kohlenstoff-Atomen sein oder bilden können;
R³ und R⁴ Alkyl-Gruppen mit 1 bis 18 Kohlenstoff-Atomen sind;
B eine Alkyliden-Gruppe der Formel 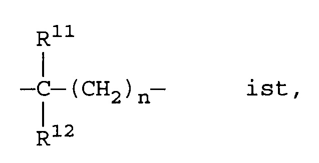 worin
n 1 bis 8 ist und
R¹¹ und R¹² Wasserstoff oder Alkyl-Gruppen mit 1 bis 8 Kohlenstoff-Atomen sind;
R¹⁰ Wasserstoff, Alkyl-Gruppen mit 1 bis 18 Kohlenstoff-Atomen, Acyl, 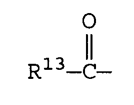 , worin
R¹³ Wasserstoff, Alkyl oder Alkenyl mit bis zu 18 Kohlenstoff-Atomen, Phenyl oder Naphthyl ist,
O oder OH ist und
Y H₂ oder O ist,
mit der Maßgabe daß R¹¹ und R¹² nicht beide Wasserstoff sind.

2. Verbindungen nach Anspruch 1, worin
R¹ Alkyl mit 1 bis 8 Kohlenstoff-Atomen ist,
R² Alkyl mit 1 bis 6 Kohlenstoff-Atomen ist und
wenigstens eine der Gruppen R¹ und R² eine t-Alkyl-Gruppe ist,
R³ und R⁴ Alkyl-Gruppen mit 1 bis 8 Kohlenstoff-Atomen sind;
R⁵ Wasserstoff oder Alkyl mit 1 bis 6 Kohlenstoff-Atomen ist;
R⁶ und R⁷ und R⁸ und R⁹ Alkyl mit 1 bis 6 Kohlenstoff-Atomen oder Ringe mit 5 bis 7 Kohlenstoff-Atomen sind;
in B n 1 bis 3 ist und R¹¹ und R¹² Wasserstoff oder Methyl sind;
R¹⁰ Wasserstoff, Alkyl-Reste mit 1 bis 18 Kohlenstoff-Atomen, O oder OH ist;
mit der Maßgabe daß R¹¹ und R¹² nicht beide Wasserstoff sind.

3. N-Alkyl-N-(3,3,5,5-tetraalkyl-2-keto-1-piperazinyl)-α-(3,5-dialkyl-4-hydroxyphenyl-α,α-dialkylacetamide nach Anspruch 2, worin R³ und R⁴ 1 bis 4 Kohlenstoff-Atome enthalten.

4. Acetamide nach Anspruch 2, worin wenigstens einer der Substituenten R¹ und R² t-Butyl oder t-Amyl ist.

5. Verbindungen nach den Ansprüchen 1 bis 4, ausgewählt aus
N-[1-(2-Keto-3,3,5,5-tetramethyl-1-piperazinyl-2-methyl-2-propyl]-α-(3,5-di-t-butyl-4-hydroxyphenyl)-2-methylpropanamid,
N-[1-(2-Keto-3,5,5-trimethyl-3-ethyl-1-piperazinyl-2-methyl-2-propyl]-α-(3,5-di-t-butyl-4-hydroxyphenyl)-2-methylpropanamid.

6. Verbindungen, ausgewählt aus
N-[1-(2-Keto-3,3-pentamethylen-5,5-dimethyl-1-piperazinyl-2-methyl-2-propyl]-α-(3,5-di-t-butyl-4-hydroxyphenyl)cyclohexancarboxamid,
N-[1-(2-Keto-3,3,5,5-tetramethyl-1-piperazinyl-2-methyl-2-propyl]-α-(3,5-di-t-butyl-4-hydroxyphenyl)cyclohexancarboxamid,
N-Cyclohexyl-N-[2-(2-keto-3,3,5,5-tetramethyl-1-piperazinyl)ethyl]-α-(3,5-di-t-butyl-4-hydroxyphenyl)cyclohexancarboxamid,
N-Cyclohexyl-N-[3-(2-keto-3,3,5,5-tetramethyl-1-piperazinyl)propyl]-α-(3,5-di-t-butyl-4-hydroxyphenyl)cyclohexancarboxamid.

7. Zusammensetzung, umfassend
(1) gegen Abbau anfälliges organisches Material und stabilisierende Mengen
(2) Verbindungen nach den Ansprüchen 1 bis 6.

8. Zusammensetzung nach Anspruch 7, worin
(1) das organische Material ein Polymer ist und
(2) aus der aus
N-[1-(2-Keto-3,3,5,5-tetramethyl-1-piperazinyl-2-methyl-2-propyl]-α-(3,5-di-t-butyl-4-hydroxyphenyl)-2-methylpropanamid,
N-[1-(2-Keto-3,5,5-trimethyl-3-ethylpiperazinyl-2-methyl-2-propyl]-α-(3,5-di-t-butyl-4-hydroxyphenyl)-2-methylpropanamid,
N-[1-(2-Keto-3,3-pentamethylen-5,5-dimethyl-1-piperazinyl2-methyl-2-propyl]-α-(3,5-di-t-butyl-4-hydroxyphenyl)cyclohexancarboxamid,
N-[1-(2-Keto-3,3,5,5-tetramethyl-1-piperazinyl-2-methyl-2-propyl]-(3,5-di-t-butyl-4-hydroxyphenyl)cyclohexancarboxamid,
N-Cyclohexyl-N-[2-(2-keto-3,3,5,5-tetramethyl-1-piperazinyl)ethyl]-α-(3,5-di-t-butyl-4-hydroxyphenyl)cyclohexancarboxamid,
N-Cyclohexyl-N-[3-(2-keto-3,3,5,5-tetramethyl-1-piperazinyl)propyl]-α-(3,5-di-t-butyl-4-hydroxyphenyl)cyclohexancarboxamid
bestehenden Gruppe ausgewählt ist.

9. Zusammensetzung nach Anspruch 8, worin das Polymer ein Olefin-Polymer ist.

10. Verfahren zur Herstellung N-(substituierter)-1-(Piperazinalkyl)-α-(3,5-dialkyl-4-hydroxyphepyl)-α,α-dialkylacetamide der allgemeinen Formel 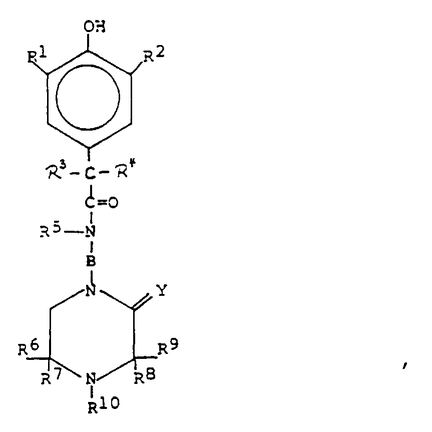 worin
R¹ und R² Alkyl-Gruppen mit 1 bis 12 Kohlenstoff-Atomen sind,
R⁵, R⁶, R⁷, R⁸ und R⁹ Wasserstoff, Alkyl-Gruppen mit 1 bis 12 Kohlenstoff-Atomen, Phenyl- und Naphthyl-Gruppen, Cycloalkyl-Gruppen mit 4 bis 12 Kohlenstoff-Atomen sind,
R⁶ und R⁷, R⁸ und R⁹ Ringe mit 5 bis 7 Kohlenstoff-Atomen sein oder bilden können;
R³ und R⁴ Alkyl-Gruppen mit 1 bis 18 Kohlenstoff-Atomen sind;
B eine Alkyliden-Gruppe der Formel 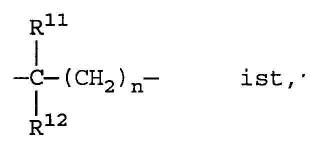 worin
n 1 bis 8 ist und
R¹¹ und R¹² Wasserstoff oder Alkyl-Gruppen mit 1 bis 8 Kohlenstoff-Atomen sind;
R¹⁰ Wasserstoff, Alkyl-Gruppen mit 1 bis 18 Kohlenstoff-Atomen, Acyl, 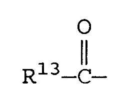 , worin
R¹³ Wasserstoff, Alkyl oder Alkenyl mit bis zu 18 Kohlenstoff-Atomen, Phenyl oder Naphthyl ist,
O oder OH ist und
Y H₂ oder O ist,
mit der Maßgabe daß R¹¹ und R¹² nicht beide Wasserstoff sind,
umfassend die gemeinsame Reaktion
eines 2,6-Dialkylphenols der Formel 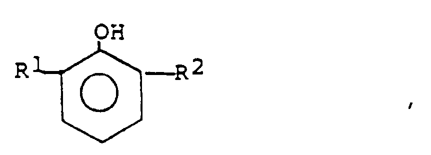 in der
R¹ und R² Alkyl-Gruppen mit bis zu 12 Kohlenstoff-Atomen sind,
eines Ketons, das aus der aus Dialkylketonen bestehenden Gruppe ausgewählt ist, worin die Alkyl-Gruppen 1 bis 18 Kohlenstoff-Atome haben,
eines aus der Chloroform und Bromform ausgewählten Haloforms und
eines Alkalimetallhydroxids und von
Piperazinen der allgemeinen Formel 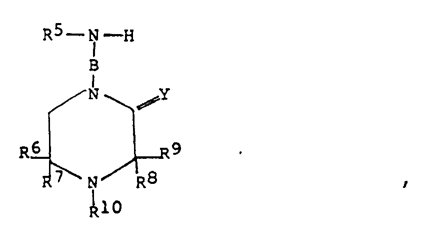 in der
R⁵ R⁶, R⁷, R⁸ und R⁹ Wasserstoff, Alkyl-Gruppen mit 1 bis 12 Kohlenstoff-Atomen, Phenyl- und Naphthyl-Gruppen, Cycloalkyl-Gruppen mit 4 bis 12 Kohlenstoff-Atomen sind, und
R⁶ und R⁷, R⁸ und R⁹ Ringe mit 5 bis 7 Kohlenstoff-Atomen sein oder bilden können;
B eine Alkyliden-Gruppe der Formel 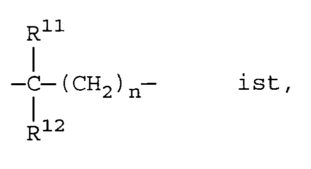 worin
n 1 bis 8 ist und
R¹¹ und R¹² Wasserstoff oder Alkyl-Gruppen mit 1 bis 8 Kohlenstoff-Atomen sind;
R¹⁰ Wasserstoff, Alkyl-Reste mit 1 bis 18 Kohlenstoff-Atomen, Acyl, 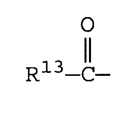 , worin
R¹³ Wasserstoff, Alkyl oder Alkenyl mit bis zu 18 Kohlenstoff-Atomen, Phenyl oder Naphthyl ist,
O oder OH ist und
Y H₂ oder O ist,
mit der Maßgabe daß R¹¹ und R¹² nicht beide Wasserstoff sind.

11. Verfahren nach Anspruch 10, worin
R¹ 1 bis 8 Kohlenstoff-Atome enthält und
R² 1 bis 6 Kohlenstoff-Atome enthält,
das Keton ein Dialkylketon der Formel 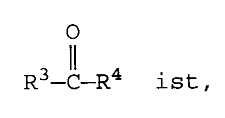 worin R³ und R⁴ Alkyl-Reste mit 1 bis 8 Kohlenstoff-Atomen sind;
R⁵ Wasserstoff oder Alkyl mit 1 bis 6 Kohlenstoff-Atomen ist;
R⁶ und R⁷ und R⁸ und R⁹ 1 bis 8 Kohlenstoff-Atome enthalten;
R¹⁰ Wasserstoff ist;
in B n 1 bis 3 ist und
R¹¹ und R¹² Wasserstoff oder Methyl sind; und
Y Sauerstoff ist;
mit der Maßgabe daß R¹¹ und R¹² nicht beide Wasserstoff sind.

## Patentansprüche (Patentansprüche für folgende(n) Vertragsstaat(en): AT, ES)

1. Verfahren zur Herstellung N-(substituierter)-1-(Piperazinalkyl)-α-(3,5-dialkyl-4-hydroxyphenyl)-α,α-dialkylacetamide der allgemeinen Formel 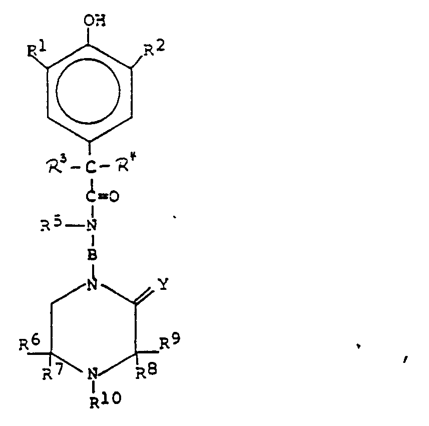 worin
R¹ und R² Alkyl-Gruppen mit 1 bis 12 Kohlenstoff-Atomen sind,
R⁵, R⁶, R⁷, R⁸ und R⁹ Wasserstoff, Alkyl-Gruppen mit 1 bis 12 Kohlenstoff-Atomen, Phenyl- und Naphthyl-Gruppen, Cycloalkyl-Gruppen mit 4 bis 12 Kohlenstoff-Atomen sind,
R⁶ und R⁷, R⁸ und R⁹ Ringe mit 5 bis 7 Kohlenstoff-Atomen sein oder bilden können;
R³ und R⁴ Alkyl-Gruppen mit 1 bis 18 Kohlenstoff-Atomen sind;
B eine Alkyliden-Gruppe der Formel 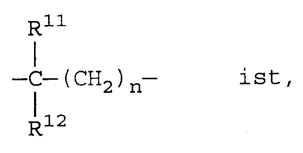 worin
n 1 bis 8 ist und
R¹¹ und R¹² Wasserstoff oder Alkyl-Gruppen mit 1 bis 8 Kohlenstoff-Atomen sind;
R¹⁰ Wasserstoff, Alkyl-Gruppen mit 1 bis 18 Kohlenstoff-Atomen, Acyl, 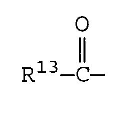 , worin
R¹³ Wasserstoff, Alkyl oder Alkenyl mit bis zu 18 Kohlenstoff-Atomen, Phenyl oder Naphthyl ist,
O oder OH ist und
Y H₂ oder O ist,
mit der Maßgabe daß R¹¹ und R¹² nicht beide Wasserstoff sind,
umfassend die gemeinsame Reaktion
eines 2,6-Dialkylphenols der Formel 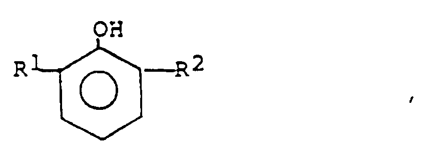 in der
R¹ und R² Alkyl-Gruppen mit 1 bis 12 Kohlenstoff-Atomen sind,
eines Ketons, das aus der aus Dialkylketonen bestehenden Gruppe ausgewählt ist, worin die Alkyl-Gruppen 1 bis 18 Kohlenstoff-Atome haben,
eines aus der Chloroform und Bromform ausgewählten Haloforms und
eines Alkalimetallhydroxids und von
Piperazinen der allgemeinen Formel 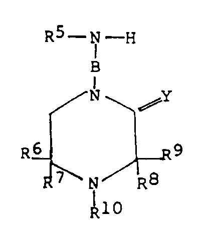
in der
R⁵ R⁶, R⁷, R⁸ und R⁹ Wasserstoff, Alkyl-Gruppen mit 1 bis 12 Kohlenstoff-Atomen, Phenyl- und Naphthyl-Gruppen, Cycloalkyl-Gruppen mit 4 bis 12 Kohlenstoff-Atomen sind, und
R⁶ und R⁷, R⁸ und R⁹ Ringe mit 5 bis 7 Kohlenstoff-Atomen sein oder bilden können;
B eine Alkyliden-Gruppe der Formel 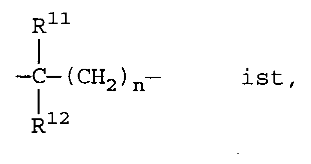 worin
n 1 bis 8 ist und
R¹¹ und R¹² Wasserstoff oder Alkyl-Gruppen mit 1 bis 8 Kohlenstoff-Atomen sind;
R¹⁰ Wasserstoff, Alkyl-Reste mit 1 bis 18 Kohlenstoff-Atomen, Acyl, 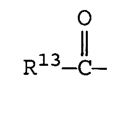 , worin
R¹³ Wasserstoff, Alkyl oder Alkenyl mit bis zu 18 Kohlenstoff-Atomen, Phenyl oder Naphthyl ist,
O oder OH ist und
Y H₂ oder O ist,
mit der Maßgabe daß R¹¹ und R¹² nicht beide Wasserstoff sind.

2. Verfahren nach Anspruch 1, worin
R¹ 1 bis 8 Kohlenstoff-Atome enthält und
R² 1 bis 6 Kohlenstoff-Atome enthält, das Keton ein Dialkylketon der Formel 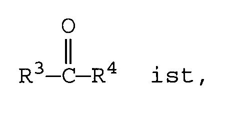 worin R³ und R⁴ Alkyl-Reste mit 1 bis 8 Kohlenstoff-Atomen sind;
R⁵ Wasserstoff oder Alkyl mit 1 bis 6 Kohlenstoff-Atomen ist;
R⁶ und R⁷ und R⁸ und R⁹ 1 bis 8 Kohlenstoff-Atome enthalten;
R¹⁰ Wasserstoff ist;
in B n 1 bis 3 ist und
R¹¹ und R¹² Wasserstoff oder Methyl sind; und
Y Sauerstoff ist;
mit der Maßgabe daß R¹¹ und R¹² nicht beide Wasserstoff sind.

3. Verfahren nach Anspruch 2, worin R³ und R⁴ 1 bis 4 Kohlenstoff-Atome enthalten.

4. Verfahren nach Anspruch 2, worin wenigstens einer der Substituenten R¹ und R² t-Butyl oder t-Amyl ist.

5. Verfahren nach den Ansprüchen 1 bis 4 zur Herstellung von Verbindungen, ausgewählt aus
N-[1-(2-Keto-3,3,5,5-tetramethyl-1-piperazinyl-2-methyl-2-propyl]-α-(3,5-di-t-butyl-4-hydroxyphenyl)-2-methylpropanamid,
N-[1-(2-Keto-3,5,5-trimethyl-3-ethyl-1-piperazinyl-2-methyl-2-propyl]-α-(3,5-di-t-butyl-4-hydroxyphenyl)-2-methylpropanamid.

6. Verfahren nach den Ansprüchen 1 bis 4 zur Herstellung von Verbindungen, ausgewählt aus
N-[1-(2-Keto-3,3-pentamethylen-5,5-dimethyl-1-piperazinyl-2-methyl-2-propyl]-α-(3,5-di-t-butyl-4-hydroxyphenyl)cyclohexancarboxamid,
N-[1-(2-Keto-3,3,5,5-tetramethyl-1-piperazinyl-2-methyl-2-propyl]-α-(3,5-di-t-butyl-4-hydroxvphenyl)cyclohexancarboxamid,
N-Cyclohexyl-N-[2-(2-keto-3,3,5,5-tetramethyl-1-piperazinyl)ethyl]-α-(3,5-di-t-butyl-4-hydroxyphenyl)cyclohexancarboxamid,
N-Cyclohexyl-N-[3-(2-keto-3,3,5,5-tetramethyl-1-piperazinyl)propyl]-α-(3,5-di-t-butyl-4-hydroxyphenyl)cyclohexancarboxamid,
wobei das Dialkylketon durch Cyclohexanon ersetzt ist.

7. Zusammensetzung, umfassend
(1) gegen Abbau anfälliges organisches Material und stabilisierende Mengen
(2) Verbindungen, die nach den Ansprüchen 1 bis 6 erhältlich sind.

8. Zusammensetzung nach Anspruch 7, worin
(1) das organische Material ein Polymer ist und
(2) aus der aus
N-[1-(2-Keto-3,3,5,5-tetramethyl-1-piperazinyl-2-methyl-2-propyl]-α-(3,5-di-t-butyl-4-hydroxyphenyl)-2-methylpropanamid,
N-[1-(2-Keto-3,5,5-trimethyl-3-ethylpiperazinyl-2-methyl-2-propyl]-α-(3,5-di-t-butyl-4-hydroxyphenyl)-2-methylpropanamid,
N-[1-(2-Keto-3,3-pentamethylen-5,5-dimethyl-1-piperazinyl-2-methyl-2-propyl]-α-(3,5-di-t-butyl-4-hydroxyphenyl)cyclohexancarboxamid,
N-[1-(2-Keto-3,3,5,5-tetramethyl-1-piperazinyl-2-methyl-2-propyl]-α-(3,5-di-t-butyl-4-hydroxyphenyl)cyclohexancarboxamid,
N-Cyclohexyl-N-[2-(2-keto-3,3,5,5-tetramethyl-1-piperazinyl)ethyl]-α-(3,5-di-t-butyl-4-hydroxyphenyl)cyclohexancarboxamid,
N-Cyclohexyl-N-[3-(2-keto-3,3,5,5-tetramethyl-1-piperazinyl)propyl]-α-(3,5-di-t-butyl-4-hydroxyphenyl)cyclohexancarboxamid
bestehenden Gruppe ausgewählt ist.

9. Zusammensetzung nach Anspruch 8, worin das Polymer ein Olefin-Polymer ist.

## Revendications (Revendications pour l'(les) Etat(s) contractant(s) suivant(s): BE, CH, DE, FR, GB, GR, IT, LI, LU, NL, SE)

1. Alpha-(3,5-di-alkyl-4-hydroxyphényl)-α,α-dialkylacétamides N-substitués par une 1-alkyl-pipérazine ayant la formule générale 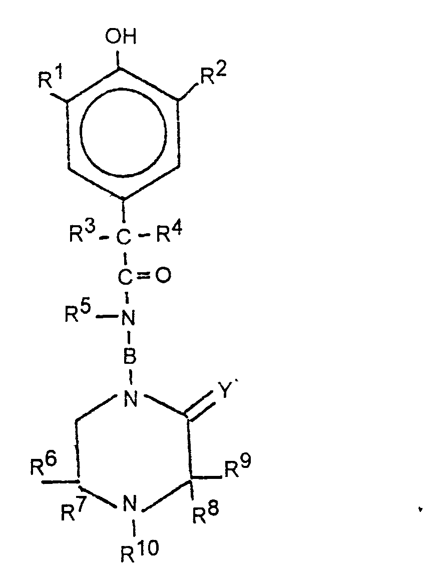 dans laquelle R¹ et R² sont des groupes alkyles contenant 1 à 12 atomes de carbone,
R⁵, R⁶, R⁷, R⁸ et R⁹ représentent un atome d'hydrogène, des groupes alkyles contenant 1 à 12 atomes de carbone, des groupes phényles et naphtyles, des groupes cycloalkyles contenant 4 à 12 atomes de carbone,
R⁶ et R⁷, R⁸ et R⁹ peuvent être ou former des cycles contenant 5 à 7 atomes de carbone;
R³ et R⁴ sont des groupes alkyles contenant 1 à 18 atomes de carbone;
B est un groupe alkylidène de formule 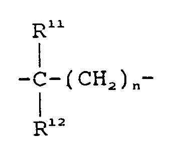 dans laquelle n vaut 1 à 8, R¹¹ et R¹² représentent un atome d'hydrogène ou des groupes alkyles contenant 1 à 8 atomes de carbone; R¹⁰ représente un atome d'hydrogène, des radicaux alkyles contenant 1 à 18 atomes de carbone, un groupe acyle 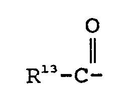 dans lequel R¹³ représente un atome d'hydrogène, un groupe alkyle ou alcényle contenant jusqu'à 18 atomes de carbone, un groupe phényle ou naphtyle, O ou OH; et Y est H₂ ou O à condition que R¹¹ et R¹² ne soient pas tous deux un atome d'hydrogène.

2. Composés selon la revendication 1, dans lequel R¹ est un groupe alkyle contenant 1 à 8 atomes de carbone, R² est un groupe alkyle contenant 1 à 6 atomes de carbone, et au moins un de R¹ et R² est un groupe t-alkyle R³ et R⁴ sont des groupes alkyles contenant 1 à 8 atomes de carbone;
R⁵ représente un atome d'hydrogène ou un groupe alkyle contenant 1 à 6 atomes de carbone; R⁶ et R⁷, R⁸ et R⁹ sont un groupe alkyle contenant 1 à 6 atomes de carbone ou des cycles contenant 5 à 7 atomes de carbone; dans B, n vaut 1 à 3 et R¹¹ et R¹² représentent un atome d'hydrogène ou un groupe méthyle; R¹⁰ représente un atome d'hydrogène ou des radicaux alkyles contenant 1 à 18 atomes de carbone, O ou OH; et Y est H₂ ou O, à condition que R¹¹ et R¹² ne soient pas tous deux un atome d'hydrogène.

3. N-(alkyl)-N-(3,3,5,5-tétraalkyl-2-céto-1-pipérazinyl)-α-(3,5-di-alkyl-4-hydroxyphényl)-α,α-dialkylacétamides selon la revendication 2, dans lesquels R³ et R⁴ contiennent 1 à 4 atomes de carbone.

4. Acétamides selon la revendication 2, dans lesquels au moins un de R¹ et R² est un groupe t-butyle ou t-amyle.

5. Composés selon les revendications 1 à 4 choisis parmi:
le N-[1-(2-céto-3,3,5,5-tétraméthyl-1-pipérazinyl)-2-méthyl-2-propyl]-α-(3,5-di-t-butyl-4-hydroxyphényl-2-méthylpropanamide;
le N-[1-(2-céto-3,5,5-triméthyl-3-éthyl-1-pipérazinyl)-2-méthyl-2-propyl]-α-(3,5-di-t-butyl-4-hydroxyphényl)-2-méthylpropanamide.

6. Composés choisis parmi:
le N-[1-(2-céto-3,3-pentaméthylène-5,5-diméthyl-1-pipérazinyl)-2-méthyl-2-propyl]-α-(3,5-di-t-butyl-4-hydroxyphényl)cyclohexanecarboxamide;
le N-[1-(2-céto-3,3,5,5-tétraméthyl-1-pipérazinyl)-2-méthyl-2-propyl]-α-(3,5-di-t-butyl-4-hydroxyphényl)cyclohexanecarboxamide;
le N-cyclohexyl-N-[2-(2-céto-3,3,5,5-tétraméthyl-1-pipérazinyl)-éthyl]-α-(3,5-di-t-butyl-4-hydroxyphényl)cyclohexanecarboxamide;
le N-cyclohexyl-N-[3-(2-céto-3,3,5,5-tétraméthyl-1-piperazinyl)propyl]-α-(3,5-di-t-butyl-4-hydroxyphényl)cyclohexanecarboxamide.

7. Composition comprenant des matières organiques (1) sujettes à la dégradation et des quantités stabilisantes de composés (2) selon les revendications 1 à 6.

8. Composition selon la revendication 7, dans laquelle ladite matière organique solide (1) est un polymère et (2) est choisie dans le groupe formé par:
le N-[1-(2-céto-3,3,5,5-tétraméthyl-1-pipérazinyl)-2-méthyl-2-propyl]-α-(3,5-di-t-butyl-4-hydroxyphény)-2-méthylpropanamide;
le N-[1-(2-céto-3,5,5-triméthyl-3-éthyl-pipérazinyl)-2-méthyl-2-propyl]-α-(3,5-di-t-butyl-4-hydroxyphényl)-2-méthylpropanamide;
le N-[1-(2-céto-3,3-pentaméthylène-5,5-diméthyl-1-pipérazinyl)-2-méthyl-2-propyl]-α-(3,5-di-t-butyl-4-hydroxyphényl)cyclohexanecarboxamide;
le N-[1-(2-céto-3,3,5,5-trétraméthyl-1-pipérazinyl)-2-méthyl-2-propyl]-(3,5-di-t-butyl-4-hydroxyphényl)cyclohexanecarboxamide;
le N-cyclohexyl-N-[2-(2-céto-3,3,5,5-tétraméthyl-1-pipérazinyl)éthyl]-α-(3,5-di-t-butyl-4-hydroxyphényl)cyclohexanecarboxamide;
le N-cyclohexyl-N-[3-(2-céto-3,3,5,5-tétraméthyl-1-pipérazinyl)propyl]-α-(3,5-di-t-butyl-4-hydroxyphényl)cyclohexanecarboxamide.

9. Composition selon la revendication 8, dans laquelle le polymère est un polymère oléfine.

10. Procédé pour préparer des α-(3,5-di-alkyl-4-hydroxyphényl)-α,α-dialkylacétamides N-substitués par une 1-alkyl-pipérazine ayant la formule générale 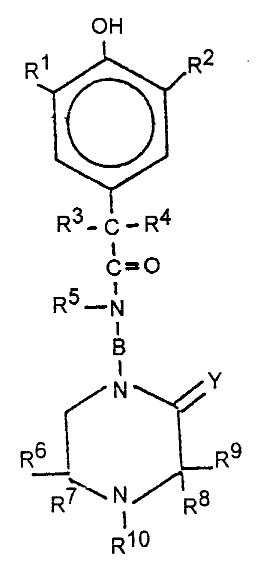 dans laquelle R¹ et R² sont des groupes alkyles contenant 1 à 12 atomes de carbone,
R⁵, R⁶, R⁷, R⁸ et R⁹ représentent un atome d'hydrogène, des groupes alkyles contenant 1 à 12 atomes de carbone, des groupes phényles et naphtyles, des groupes cycloalkyles contenant 4 à 12 atomes de carbone,
R⁶ et R⁷, R⁸ et R⁹ peuvent être ou former des cycles contenant 5 à 7 atomes de carbone;
R³ et R⁴ sont des groupes alkyles contenant 1 à 18 atomes de carbone;
B est un groupe alkylidène de formule 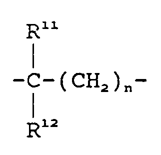 dans laquelle n vaut 1 à 8, R¹¹ et R¹² représentent un atome d'hydrogène ou des groupes alkyles contenant 1 à 8 atomes de carbone; R¹⁰ représente un atome d'hydrogène, des radicaux alkyles contenant 1 à 18 atomes de carbone, un groupe acyle 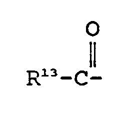 dans lequel R¹³ représente un atome d'hydrogène, un groupe alkyle ou alcényle contenant jusqu'à 18 atomes de carbone, un groupe phényle ou naphtyle, O ou OH; et Y est H₂ ou O à condition que R¹¹ et R¹² ne soient pas tous deux un atome d'hydrogène, comprenant la réaction conjointe d'un 2,6-dialkylphénol ayant la formule 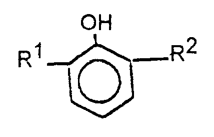 dans laquelle R¹ et R² sont des groupes alkyles contenant jusqu'à 12 atomes de carbone; d'une cétone choisie dans le groupe constitué de dialkylcétones,
dans laquelle les groupes alkyles contiennent 1 à 18 atomes de carbone, d'un halogénoforme choisi dans le groupe constitué du chloroforme et du bromoforme, et d'un hydroxyde de métal alcalin et de pipérazines, ayant la formule générale 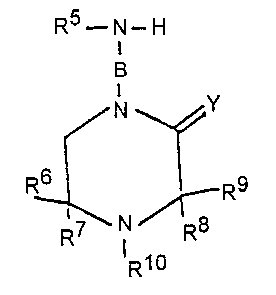 dans laquelle R⁵, R⁶, R⁷, R⁸ et R⁹ représentent des atomes d'hydrogène, des groupes alkyles contenant 1 à 12 atomes de carbone, des groupes phényles et naphtyles, des groupes cycloalkyles contenant 4 à 12 atomes de carbone, et R⁶ et R⁷, R⁸ et R⁹ peuvent être ou former des cycles contenant 5 à 7 atomes de carbone; B est un groupe alkylidène de formule 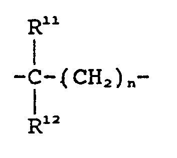 dans laquelle n vaut 1 à 8, R¹¹ et R¹² représentent un atome d'hydrogène ou des groupes alkyles contenant 1 à 8 atomes de carbone; R¹⁰ est un atome d'hydrogène, des radicaux alkyle contenant 1 à 18 atomes de carbone, un groupe acyle 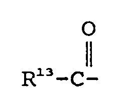 dans lequel R¹³ représente un atome d'hydrogène, un groupe alkyle ou alcényle contenant jusqu'à 18 atomes de carbone, un groupe phényle ou naphtyle, O ou OH; et Y est H₂ ou O à condition que R¹¹ et R¹² ne soient pas tous deux un atome d'hydrogène.

11. Procédé selon la revendication 10, dans lequel R¹ contient 1 à 8 atomes de carbone et R² contient 1 à 6 atomes de carbone, la cétone est une dialkylcétone de formule 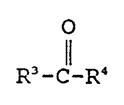 dans laquelle R³ et R⁴ sont des radicaux alkyles contenant 1 à 8 atomes de carbone;
R⁵ représente un atome d'hydrogène ou un groupe alkyle de 1 à 6 atomes de carbone, R⁶ et R⁷, R⁸ et R⁹ contiennent 1 à 8 atomes de carbone; R¹⁰ est un atome d'hydrogène; dans B, n vaut 1 à 3 et R¹¹ et R¹² représentent un atome d'hydrogène ou un groupe méthyle et Y est un atome d'oxygène, à condition que R¹¹ et R¹² ne soient pas tous deux un atome d'hydrogène.

## Revendications (Revendications pour l'(les) Etat(s) contractant(s) suivant(s): AT, ES)

1. Procédé pour préparer des α-(3,5-di-alkyl-4-hydroxyphényl)-α,α-dialkylacétamides N-substitués par une 1-alkyl-pipérazine ayant la formule générale 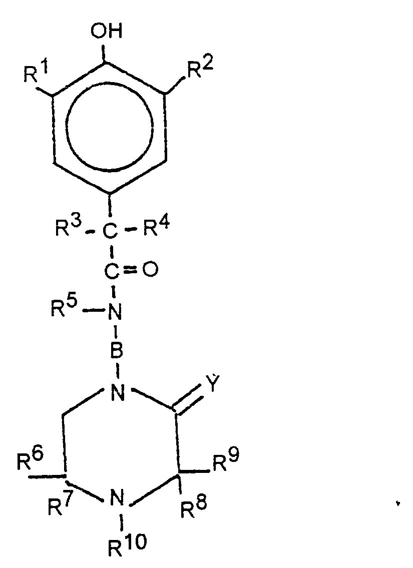 dans laquelle R¹ et R² sont des groupes alkyles contenant 1 à 12 atomes de carbone,
R⁵, R⁶, R⁷, R⁸ et R⁹ représentent un atome d'hydrogène, des groupes alkyles contenant 1 à 12 atomes de carbone, des groupes phényles et naphtyles, des groupes cycloalkyles contenant 4 à 12 atomes de carbone,
R⁶ et R⁷, R⁸ et R⁹ peuvent être ou former des cycles contenant 5 à 7 atomes de carbone;
R³ et R⁴ sont des groupes alkyles contenant 1 à 18 atomes de carbone;
B est un groupe alkylidène de formule 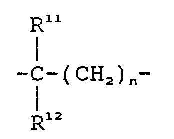 dans laquelle n vaut 1 à 8, R¹¹ et R¹² représentent un atome d'hydrogène ou des groupes alkyles contenant 1 à 8 atomes de carbone; R¹⁰ représente un atome d'hydrogène, des radicaux alkyles contenant 1 à 18 atomes de carbone, un groupe acyle 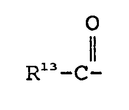 dans lequel R¹³ représente un atome d'hydrogène, un groupe alkyle ou alcényle contenant jusqu'à 18 atomes de carbone, un groupe phényle ou naphtyle, O ou OH; et Y est H₂ ou O à condition que R¹¹ et R¹² ne soient pas tous deux un atome d'hydrogène, comprenant la réaction conjointe d'un 2,6-dialkylphénol ayant la formule 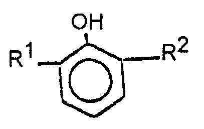 dans laquelle R¹ et R² sont des groupes alkyles contenant jusqu'à 12 atomes de carbone; d'une cétone choisie dans le groupe constitué de dialkylcétones,
dans laquelle les groupes alkyles contiennent 1 à 18 atomes de carbone, d'un halogénoforme choisi dans le groupe constitué du chloroforme et du bromoforme, et d'un hydroxyde de métal alcalin et de pipérazines, ayant la formule générale 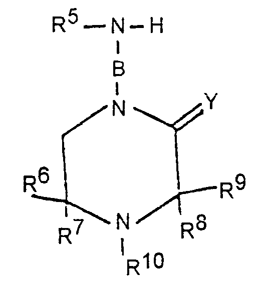 dans laquelle R⁵, R⁶, R⁷, R⁸ et R⁹ représentent des atomes d'hydrogène, des groupes alkyles contenant 1 à 12 atomes de carbone, des groupes phényles et naphtyles, des groupes cycloalkyles contenant 4 à 12 atomes de carbone, et R⁶ et R⁷, R⁸ et R⁹ peuvent être ou former des cycles contenant 5 à 7 atomes de carbone; B est un groupe alkylidène de formule 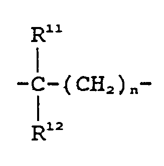 dans laquelle n vaut 1 à 8, R¹¹ et R¹² représentent un atome d'hydrogène ou des groupes alkyles contenant 1 à 8 atomes de carbone; R¹⁰ est un atome d'hydrogène, des radicaux alkyles contenant 1 à 18 atomes de carbone, un groupe acyle 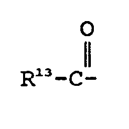 dans lequel R¹³ représente un atome d'hydrogène, un groupe alkyle ou alcényle contenant jusqu'à 18 atomes de carbone, un groupe phényle ou naphtyle, O ou OH; et Y est H₂ ou O à condition que R¹¹ et R¹² ne soient pas tous deux un atome d'hydrogène.

2. Procédé selon la revendication 1, dans lequel R¹ contient 1 à 8 atomes de carbone et R² contient 1 à 6 atomes de carbone, la cétone est une dialkylcétone de formule 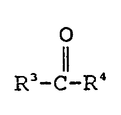 dans laquelle R³ et R⁴ sont des radicaux alkyles contenant 1 à 8 atomes de carbone;
R⁵ représente un atome d'hydrogène ou un groupe alkyle de 1 à 6 atomes de carbone, R⁶ et R⁷, R⁸ et R⁹ contiennent 1 à 8 atomes de carbone; R¹⁰ est un atome d'hydrogène; dans B, n vaut 1 à 3 et R¹¹ et R¹² représentent un atone d'hydrogène ou un groupe méthyle et Y est un atome d'oxygène, à condition que R¹¹ et R¹² ne soient pas tous deux un atome d'hydrogène.

3. Procédé selon la revendication 2, dans lequel R³ et R⁴ contiennent 1 à 4 atomes de carbone.

4. Procédé selon la revendication 2, dans lequel au moins un de R¹ et R² est un groupe t-butyle ou t-amyle.

5. Procédé selon les revendications 1 à 4 pour préparer des composés choisis parmi:
le N-[1-(2-céto-3,3,5,5-tétraméthyl-1-pipérazinyl)-2-méthyl-2-propyl]-α-(3,5-di-t-butyl-4-hydroxyphényl)-2-méthylpropanamide;
le N-[1-(2-céto-3,5,5-triméthyl-3-éthyl-1-pipérazinyl)-2-méthyl-2-propyl]-α-(3,5-di-t-butyl-4-hydroxyphényl)-2-méthylpropanamide.

6. Procédé selon les revendications 1 à 4 pour préparer les composés choisis parmi:
le N-[1-(2-céto-3,3-pentaméthylène-5,5-diméthyl-1-pipérazinyl)-2-méthyl-2-propyl]-α-(3,5-di-t-butyl-4-hydroxyphényl)cyclohexanecarboxamide;
le N-[1-(2-céto-3,3,5,5-tétraméthyl-1-pipérazinyl)-2-méthyl-2-propyl]-α-(3,5-di-t-butyl-4-hydroxyphényl)cyclohexanecarboxamide;
le N-cyclohexyl-N-[2-(2-céto-3,3,5,5-tétraméthyl-1-pipérazinyl)-éthyl]-α-(3,5-di-t-butyl-4-hydroxyphényl)cyclohexanecarboxamide;
le N-cyclohexyl-N-[3-(2-céto-3,3,5,5-tétraméthyl-1-pipérazinyl)propyl]-α-(3,5-di-t-butyl-4-hydroxyphényl)cyclohexanecarboxamide, par ce moyen la dialkylcétone est substituée par la cyclohexanone.

7. Composition comprenant des matières organiques (1) sujettes à la dégradation et des quantités stabilisantes de composés (2) que l'on peut obtenir selon les revendications 1 à 6.

8. Composition selon la revendication 7, dans laquelle ladite matière organique solide (1) est un polymère et (2) est choisie dans le groupe formé par:
le N-[1-(2-céto-3,3,5,5-tétraméthyl-1-pipérazinyl)-2-méthyl-2-propyl]-α-(3,5-di-t-butyl-4-hydroxyphényl)-2-méthylpropanamide;
le N-[1-(2-céto-3,5,5-triméthyl-3-éthyl-pipérazinyl)-2-méthyl-2-propyl]-α-(3,5-di-t-butyl-4-hydroxyphényl)-2-méthylpropanamide;
le N-[1-(2-céto-3,3-pentaméthylène-5,5-diméthyl-1-pipérazinyl)-2-méthyl-2-propyl]-α-(3,5-di-t-butyl-4-hydroxyphényl)cyclohexanecarboxamide;
le N-[1-(2-céto-3,3,5,5-trétraméthyl-1-pipérazinyl)-2-méthyl-2-propyl]-(3,5-di-t-butyl-4-hydroxyphényl)cyclohexane carboxamide;
le N-cyclohexyl-N-[2-(2-céto-3,3,5,5-tétramethyl-1-pipérazinyl)éthyl]-α-(3,5-di-t-butyl-4-hydroxyphényl)cyclohexanecarboxamide;
le N-cyclohexyl-N-[3-(2-céto-3,3,5,5-tétraméthyl-1-pipérazinyl)propy]-α-(3,5-di-t-butyl-4-hydroxyphény)cyclohexanecarboxamide.

9. Composition selon la revendication 8, dans laquelle le polymère est un polymère oléfine.
